(19) Europäisches Patentamt / European Patent Office / Office européen des brevets

(11) **EP 4 487 877 A1**

(12) **DEMANDE DE BREVET EUROPEEN**

(43) Date de publication:
**08.01.2025 Bulletin 2025/02**

(21) Numéro de dépôt: **24186518.7**

(22) Date de dépôt: **04.07.2024**

(51) Classification Internationale des Brevets (IPC):
**A61L 27/20** (2006.01)      **A61L 27/52** (2006.01)
**A61L 27/54** (2006.01)      **A61L 27/58** (2006.01)

(52) Classification Coopérative des Brevets (CPC):
(C-Sets disponibles)
**A61L 27/52; A61L 27/20; A61L 27/54; A61L 27/58;**
A61K 9/0024; A61K 47/32; A61K 47/36;
A61L 2300/62                                      (Cont.)

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **06.07.2023  FR 2307213**

(71) Demandeur: **Commissariat à l'Energie Atomique et aux Energies Alternatives**
**75015 Paris (FR)**

(72) Inventeurs:
• **TEXIER-NOGUES, Isabelle**
  **38054 GRENOBLE CEDEX 09 (FR)**
• **HOANG, Antoine**
  **38054 GRENOBLE CEDEX 09 (FR)**
• **BOTTAUSCI, Frédéric**
  **38054 GRENOBLE CEDEX 09 (FR)**

(74) Mandataire: **Cabinet Nony**
**11 rue Saint-Georges**
**75009 Paris (FR)**

(54) **PROCÉDÉ DE SYNTHÈSE D'HYDROGELS DÉGRADABLES**

(57)    La présente invention concerne à titre principal un procédé de préparation d'un hydrogel réticulé à dégradabilité contrôlée comprenant au moins les étapes consistant à

(a) Disposer de gouttelettes aqueuses comprenant au moins un mélange d'au moins deux biopolymères réticulables et de degrés de substitution, DS différents,

(b) Exposer lesdites gouttelettes à un stimuli de réticulation efficace pour réaliser la réticulation simultanée des deux biopolymères et,

(c) Récupérer l'hydrogel réticulé,

dans lequel l'un des biopolymères possède un degré de substitution inférieur ou égal à 0,2 Figure pour l'abrégé : néant

**EP 4 487 877 A1**

(52) Classification Coopérative des Brevets (CPC):
(Cont.)

C-Sets
**A61L 27/20, C08L 5/02**

**EP 4 487 877 A1**

**Description**

**Domaine technique**

La présente invention a trait au domaine des hydrogels utiles pour encapsuler et délivrer des entités d'intérêt, chimiques comme par exemple des actifs thérapeutiques ou des réactifs tel que par exemple un catalyseur, ou des entités d'intérêt biologiques, comme par exemple des biomolécules, acides nucléiques, protéines ou anticorps, cellules, ou virus et bactéries à visée thérapeutique.

Plus particulièrement elle vise à proposer un procédé de préparation d'hydrogels aptes à libérer de manière contrôlée la ou les entités d'intérêt qu'ils encapsulent.

**Technique antérieure**

Les hydrogels, par définition, sont des réseaux tridimensionnels réticulés, insolubles dans l'eau compte-tenu de leur réticulation, et qui sont capables d'absorber et de retenir d'importantes quantités d'un milieu aqueux, comme de l'eau voire un fluide biologique. Un hydrogel peut être notamment classé au regard de ses dimensions et ses formats en tant que macrogel, microgel voire nanogel.

Des microgels réticulés à base de différents polymères, en particulier de biopolymères tels que l'alginate, le chitosan, l'acide hyaluronique, et en particulier le dextran ont déjà été décrits pour des applications d'encapsulation et de libération d'entités d'intérêt chimiques ou biologiques.

Ces biopolymères offrent en effet plusieurs avantages parmi lesquels des propriétés "bio" comme une synthèse naturelle dans des milieux physiologiques, des propriétés de biodégradabilité par voie enzymatique et une sensibilité à des stimuli externes tels que la température, le pH, la force ionique et/ou la polarité. Ils peuvent être nativement réticulables à l'image par exemple de l'alginate doté de fonctions carboxyliques, ou aisément chimiquement modifiés pour devenir réticulables comme par exemple un dextran, chimiquement modifié pour être substitué par des fonctions réticulables comme par exemple méthacrylates.

La mise en oeuvre d'hydrogels réticulés de biopolymères à des fins d'encapsulation et relargage contrôlé d'entités d'intérêt est donc toute particulièrement intéressante au regard des propriétés précitées sous réserve de pouvoir contrôler leurs stabilité et dégradabilité. Il importe en effet que ces hydrogels aient un degré de réticulation suffisant pour prévenir leur déstabilisation à des stimuli externes mais néanmoins compatible avec une dégradation en particulier par voie enzymatique pour libérer la ou les entités d'intérêt encapsulée(s).

Les méthodes classiques décrites dans la littérature pour ajuster les propriétés de solidité (i.e. propriétés mécaniques) et de dégradabilité d'un hydrogel reposent généralement sur l'ajustement de son poids moléculaire, sa concentration lors de la réaction de réticulation, ou du degré de substitution, DS, du biopolymère dont il dérive. Toutefois, ces méthodes ne s'avèrent pas totalement efficaces pour atteindre ce compromis entre stabilité et dégradabilité enzymatique. Ainsi, des études réalisées sur des nanogels de dextran montrent que si le polymère dextran est réticulé à une échelle élevée, la dextranase ne peut venir digérer le nanogel. En outre, le contrôle du processus de libération des entités encapsulées, en termes de durée, entre 2 et 3 heures notamment, soulève une difficulté complémentaire.

**Exposé de l'invention**

La présente invention a précisément pour objectif la mise au point d'un hydrogel de biopolymères répondant à ces attentes.

En particulier, elle vise à proposer un hydrogel réticulé efficace pour l'encapsulation temporaire d'une ou plusieurs entités d'intérêt.

Elle vise également à proposer un hydrogel réticulé stabilisé à l'égard de stimuli externes comme en particulier la température, le pH et les forces ioniques.

Elle vise aussi à proposer un hydrogel réticulé dégradable par voie enzymatique.

Elle vise également à proposer un hydrogel réticulé dont la biodégradabilité enzymatique est contrôlée en termes de durée et notamment est non immédiate mais achevée en moins de 3 heures et en particulier en moins de 2 heures.

Ainsi, selon l'un de ses aspects, la présente invention concerne un procédé de préparation d'un hydrogel réticulé à dégradabilité contrôlée comprenant au moins les étapes consistant à

(a) Disposer de gouttelettes aqueuses comprenant au moins un mélange d'au moins deux biopolymères réticulables et de degré de substitution DS différents,
(b) Exposer lesdites gouttelettes à un stimuli de réticulation efficace pour réaliser la réticulation simultanée des deux biopolymères et
(c) Récupérer l'hydrogel réticulé,

3

dans lequel l'un des biopolymères possède un degré de substitution, DS, inférieur ou égal à 20%.

Conventionnellement, le DS correspond au nombre de fonctions polymérisables pour 100 unités monomères. Ce DS est déterminable par 1H RMN notamment comme détaillé pour un biopolymère spécifique de dextran dans .

Contre toute attente et comme il ressort des exemples figurant ci-après, les inventeurs ont constaté que la réticulation simultanée d'au moins deux biopolymères réticulables possédant des degrés de substitution, DS, différents avec seul l'un d'entre eux possédant un DS faible et en particulier inférieur ou égal à 20%, permet d'atteindre le compromis recherché à savoir une stabilité aux stimuli connus pour précisément déstabiliser ces biopolymères et une biodégradabilité néanmoins possible sur une durée contrôlée notamment de l'ordre de moins de 3 heures voire moins de 2 heures.

Selon un mode de réalisation particulier, les biopolymères considérés ne se distinguent l'un de l'autre que par leur degré de substitution, DS.

En particulier, seul l'un des biopolymères du mélange possède un DS inférieur ou égal à 20%.

En particulier, les biopolymères sont chimiquement modifiés et notamment substitués par des motifs réticulables notamment choisis parmi les motifs thiol, divinylsulfonyle, maléimide, azoture, alcynyle, alcènyle, acrylate, méthacrylate, aldéhyde, norbornènyle, oxy-amine, amine, ester N-hydroxysuccinimide et leurs mélanges, en particulier parmi les acrylates et méthacrylates.

En particulier, les gouttelettes contiennent un mélange de deux polydextrans de poids moléculaire identique et chimiquement modifiés avec des motifs choisis parmi les acrylate, méthacrylate, alcényle et alcynyle et en particulier un mélange de deux polydextrans de poids moléculaire identique chimiquement modifiés par des motifs méthacrylate, DexMet, et de DS variant de 10 à 40%.

En particulier, les deux biopolymères dérivent du dextran et notamment sont des polydextrans chimiquement modifiés par des motifs méthacrylate, dits encore Dex(Met).

Plus particulièrement, les gouttelettes contiennent un mélange d'au moins un DexMet de DS inférieur à 20% et d'un DexMet de DS supérieur à 25% et notamment un mélange d'un DexMet à DS égal à 13% +/- 0,5% et d'un DexMet à DS égal à 31% +/-1%.

Selon un autre de ses aspects, la présente invention concerne un hydrogel réticulé à dégradation contrôlée, caractérisé en ce qu'il dérive de la réticulation simultanée d'au moins deux polydextrans chimiquement modifiés avec des motifs choisis parmi les motifs thiol, divinylsulfonyle, maléimide, azoture, alcynyle, alcènyle, acrylate, méthacrylate, aldéhyde, norbornènyle, oxy-amine, amine, ester N-hydroxysuccinimide et en particulier parmi les acrylates et méthacrylates, dont l'un possédant un DS inférieur ou égal à 20%.

En particulier, les deux polydextrans ne se différencient que par leur DS et plus particulièrement sont chimiquement modifiés par des motifs méthacrylate, DexMet, et leur DS varient de 10% à 40%.

Selon un mode de réalisation particulier, cet hydrogel est obtenu par le procédé selon l'invention.

Selon un mode de réalisation particulier, cet hydrogel contient en outre au moins une entité d'intérêt à libérer de manière contrôlée.

Selon encore un autre de ses aspects, la présente invention concerne l'utilisation d'un hydrogel conforme à l'invention pour la culture cellulaire ou bactérienne.

Selon encore un autre de ses aspects, la présente invention concerne l'utilisation de gouttelettes aqueuses, notamment formées par voie microfluidique, et comprenant au moins un mélange d'au moins deux polydextrans réticulables et chimiquement modifiés avec des motifs choisis parmi les motifs thiol, divinylsulfonyle, maléimide, azoture, alcynyle, alcènyle, acrylate, méthacrylate, aldéhyde, norbornènyle, oxy-amine, amine, ester N-hydroxysuccinimide et leurs mélanges en particulier parmi les acrylates et méthacrylates avec l'un d'entre eux et en particulier un seul d'entre eux possédant un DS inférieur ou égal à 20%, pour l'encapsulation d'au moins une entité d'intérêt.

D'autres caractéristiques, variantes et avantages des objets de l'invention, ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

## Brève description des dessins

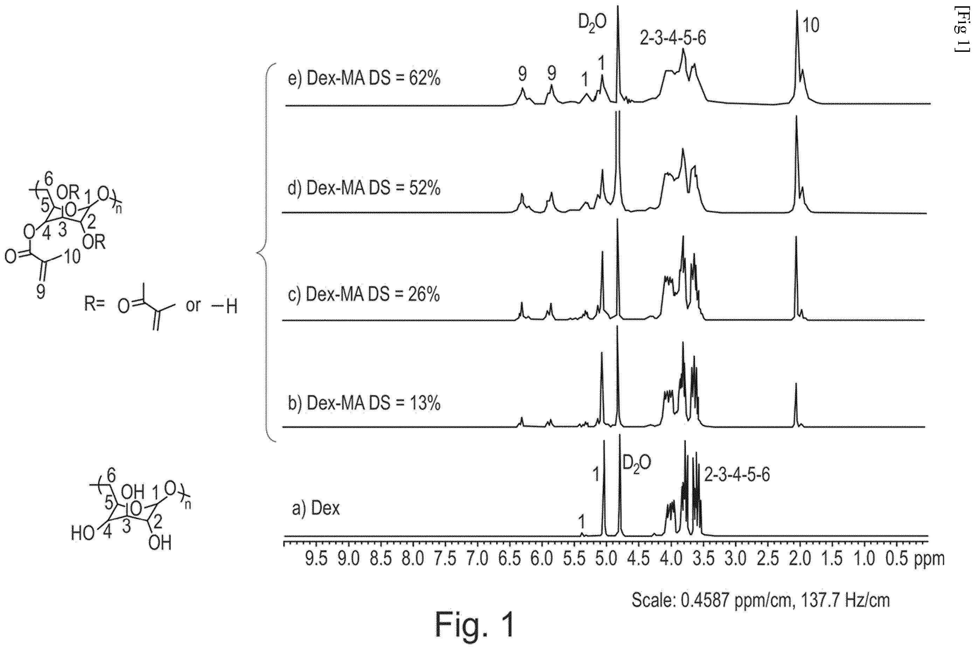

[Fig 1] : Elle représente les spectres RMN 1H du a) Dextran et b) Dextran-méthacrylate (0,0625 eq à 0.3 eq d'anhydride méthacrylique) dans D2O à 300 MHz.

## Description détaillée

Comme il ressort de ce qui précède, l'invention vise à proposer de nouveaux hydrogels dédiés notamment à encapsuler et libérer des entités d'intérêt chimique et/ou biologique et qui combinent une stabilisation à des stimuli externes et en particulier aux tensioactifs à une biodégradabilité enzymatique contrôlée car non immédiate mais à bref délai.

Par définition, le terme hydrogel désigne un réseaux tridimensionnel réticulé, insoluble dans l'eau et qui est capable d'absorber et de retenir d'énormes quantités d'un milieu aqueux, comme de l'eau voire un fluide biologique. L'hydrogel peut être un macrogel, microgel voire un nanogel. Ces réseaux colloïdaux gonflables à l'eau ont des diamètres typiques allant de 20 à 1000 nm pour les nanogels, de 1 μm à 800 μm pour les microgels, et de taille supérieure pour les macrogels.

Dans le cadre de l'invention, les hydrogels considérés sont de préférence des microgels et de diamètre typique variant de 10 à 100 μm.

Les gouttelettes peuvent être caractérisées au microscope binoculaire équipé d'une caméra pour l'acquisition d'images (10x/0.25 Olympus, tube lentille 75 mm caméra achromatique IDS UI388xCP-M). La taille des pixels composant l'image est déterminée (0.58μm/pixel) et en connaissant le diamètre d'une goutte par la mesure directe au microscope, la lecture d'une parcelle permet alors de remonter au nombre de gouttes par cm$^{-2}$.

Les hydrogels considérés selon l'invention sont formés à partir de biopolymères.

Au sens de l'invention, le terme "biopolymère" désigne un polymère dérivant de ressources naturelles renouvelables (un matériau dit encore "naturel). Les biopolymères considérés selon l'invention ont l'avantage de posséder, outre une très bonne compatibilité avec la peau humaine, une biodégradabilité en tant que tels voire in vivo.

Ce terme « biopolymère » couvre ainsi les polymères naturels tels que par exemple une protéine ou un polysaccharide mais également les dérivés de polymères naturels comme par exemple ceux qui dérivent d'une modification chimique de ces polymères naturels pour les rendre réticulables ou compatibles avec un mode de réticulation spécifique.

A titre illustratif de biopolymères convenant à l'invention, peuvent notamment être cités les polyhydroxyacides, tels que par exemple l'acide polyglycolique (PGA) et l'acide polylactique (PLA), également connu sous le nom de polylactide ; les polysaccharides, tels que par exemple la cellulose, la chitine et l'amidon et des protéines ou des peptides, tels que par exemple le collagène, la gélatine, les élastines, les fibroïnes, la zéine de maïs, la soie de diverses espèces de vers à soie, la kératine et leurs dérivés.

Comme précisé ci-dessus, ces biopolymères se doivent d'être réticulables pour former des noeuds de réticulation chimique (covalent, ionique, liaison de coordination) dans le réseau tridimensionnel constituant l'hydrogel correspondant et en outre dégradables en particulier par une enzyme.

Selon un mode de réalisation particulier, ces biopolymères dérivent de composés choisis parmi les alginates, l'acide hyaluronique, la cellulose, carboxyméthylcellulose, le chitosan, le dextran. Leurs polymères respectifs sont en effet enzymatiquement dégradables. A titre représentatif des enzymes correspondantes, peuvent être par exemple citées l'alginate lysase, la hyaluronidase, les estérases, la dextranase, la cellulase et la chitosanase.

Selon un autre mode de réalisation, ces biopolymères peuvent dériver du collagène, gélatine, élastine ou de poly(acide aminés) dégradables par des métalloprotéases.

Ces biopolymères sont réticulés dans le cadre de la présente invention. Nativement, ils peuvent être réticulables s'ils portent des motifs réticulables à l'image de fonctions carboxyliques comme par exemple l'alginate.

Dans le cas contraire, ces biopolymères sont mis en oeuvre sous une forme dites chimiquement modifiée pour les pourvoir précisément en groupements fonctionnels réticulables. Cette modification chimique consiste le plus souvent à introduire dans la structure des polymères à modifier des motifs réticulables en particulier choisis parmi les motifs thiol, divinylsulfonyle, maléimide, azoture, alcynyle, alcényle, acrylate, méthacrylate, aldéhyde, norbornènyle, oxy-amine, amine, ester N-hydroxysuccinimide et en particulier parmi les acrylates, méthacrylates.

Ces groupements fonctionnels qui permettront la réticulation, sont introduits avec un degré de substitution donné, DS.

En l'occurrence, les hydrogels selon l'invention dérivent de la réticulation simultanée d'au moins deux biopolymères réticulables et de degré de substitution différent dont un étant inférieur à 20%.

Selon un mode de réalisation particulier, les deux biopolymères réticulables ne se différencient que par leur degré de substitution, DS. En d'autres termes, ils ont la même nature chimique et le même poids moléculaire. En particulier, les deux biopolymères réticulables dérivent du dextran.

Un polydextran photoréticulable est obtenu par modification chimique d'un polydextran pour introduire des groupements chimiques photosensibles, tels que des groupements fonctionnels insaturés (acrylate, méthacrylate, alcényle, alcynyle,..). Les acrylates et les méthacrylates sont bien connus en tant que groupes photopolymérisables pour fonctionnaliser les polysaccharides car ils présentent une photoréactivité élevée pour polymériser en présence d'un photo-initiateur.

Selon un mode de réalisation préféré, les gouttelettes considérées dans le procédé selon l'invention contiennent un mélange de deux polydextrans de poids moléculaire identique et chimiquement modifiés par des motifs choisis parmi les acrylate, méthacrylate, alcényle, alcynyle et leurs mélanges.

Le procédé selon l'invention considère notamment des gouttelettes contenant un mélange de deux polydextrans de poids moléculaire identique et chimiquement modifiés par des motifs méthacrylate, DexMet, et de DS variant de 10% à 40%.

Dans le procédé de l'invention, ces biopolymères réticulables sont mis en oeuvre au sein de gouttelettes aqueuses, en particulier de microgouttes.

Selon une première variante, les gouttelettes aqueuses de biopolymères réticulables peuvent être obtenues par un procédé microfluidique. Ce mode de fabrication est particulièrement intéressant quand il est désiré contrôler la taille des gouttelettes notamment à l'état de microgouttes et le nombre d'entités d'intérêt, si présentes, par hydrogel (par exemple 1 bactérie/hydrogel), ou si les entités d'intérêt sont fragiles.

Selon une seconde variante, ces gouttelettes sont obtenues par émulsification eau dans huile. Cette technique est généralement privilégiée pour obtenir un taux de charge élevé en entités d'intérêt comme pour l'encapsulation de drogues.

Avantageusement, les gouttelettes sont formées par voie microfluidique. Le procédé microfluidique est une technique douce. De plus les procédés microfluidiques permettent d'obtenir une population très monodisperse en microgouttes.

Comme détaillé dans les exemples ci-après, ces microgouttes peuvent notamment être formées par un procédé microfluidique de flow focusing utilisant une puce en PDMS commerciale (EZ Drop (DROPKIT01)).

La taille des gouttelettes formées dans le cadre de l'invention varie donc selon la technique utilisée pour les former.

D'une manière générale, leur taille moyenne en nombre peut varier de 10 à 100 $\mu$m en particulier de 30 à 50 $\mu$m.

On obtient les microgouttes le plus souvent dans une phase huileuse ou organique (le coeur aqueux de la gouttelette encapsulant le(s) polymère(s)).

Selon un mode de réalisation particulier, le procédé de l'invention met en oeuvre des gouttelettes qui contiennent un mélange d'au moins un DexMet de DS inférieur à 20% et au moins un DexMet de DS supérieur à 25%.

Selon un autre mode de réalisation particulier, le procédé de l'invention met en oeuvre des gouttelettes qui contiennent un mélange de DexMet à DS égal à 13% +/- 0,5% et de DexMet à DS égal à 31% +/-1%.

En particulier, ces gouttelettes contiennent un mélange à 75% en poids de DexMet à DS égal 13%+/- 0,5% et 25% en poids de DexMet à DS égal à 31%+/-1%. En particulier, les deux types de DexMet ne se différencient que par leurs DS.

Selon un autre mode de réalisation particulier, le procédé de l'invention met en oeuvre des gouttelettes qui contiennent un mélange 50/50 en poids de DexMet à DS égal à 13% +/-0,5% et de DexMet à DS égal à 3 1%+/-1%. En particulier, les deux types de DexMet ne se différencient que par leurs DS.

Comme précisé ci-dessus, le procédé selon l'invention requiert que la réticulation des deux biopolymères présents dans ces gouttelettes soit réalisée de manière simultanée.

Cette réticulation peut être réalisée par des méthodes classiques et son choix est généralement conditionné par la nature des fonctions réticulables. Elle peut être ainsi chimique ou photochimique.

En raison de sa facilité d'utilisation et de sa polyvalence, la photoréticulation chimique est l'une des méthodes classiques utilisées pour réticuler les hydrogels. Par rapport à la réticulation chimique par polymérisation radicalaire des fonctions méthacrylates, la voie photochimique permet en outre un meilleur contrôle de la réaction et ne requiert pas d'initiateur chimique de polymérisation de type catalyseur potentiellement cytotoxique utilisé dans les méthodes de réticulation chimiques conventionnelles.

Ainsi, les biopolymères présents dans les gouttelettes sont réticulés de préférence par voie photochimique.

Pour se faire, les gouttelettes contiennent également au moins un agent réticulant et notamment un photo-initiateur.

Ce photoinitiateur peut notamment être choisi parmi le phenyl-2,4,6-trimethylbenzoylphosphinate de lithium, LAP, et le 1-[4-(2-Hydroxyethoxy) -phenyl]-2-hydroxy-2-methyl-1-propane-1-one notamment commercialisé sous le nom Irgacure 2959. Ainsi, la réticulation chimique des fonctions méthacrylate de deux polydextrans, Dex-Met, de DS différents et dont l'un est inférieur ou égal à 20%, peut être obtenue lorsque la gouttelette de solution aqueuse les comprenant avec un photo-initiateur hydrosoluble est exposée à la lumière UV, en général à 365 nm ou 405 nm.

Selon ce mode réalisation, la réticulation photochimique est réalisée après la fabrication des gouttelettes incorporant les deux biopolymères, le photo-initiateur et si présente(s) une ou plusieurs entités d'intérêt.

Il est à souligner que dans le cas d'une réticulation chimique, celle-ci peut être également initiée lors de la fabrication des gouttelettes et si considérée l'encapsulation de la ou des entité(s) d'intérêt dans le coeur de ces gouttelettes. Dans ce cas, la réticulation se réalise par réaction chimique entre le polymère et plus particulièrement le groupement réactionnel introduit sur le polymère avec un DS contrôlé et un réticulant, ou entre deux chaînes de polymère portant deux groupements réactionnels introduits sur le polymère avec un DS contrôlé (et capables de réagir entre eux).

Selon une variante préférée, les gouttelettes contiennent en outre au moins une entité d'intérêt chimique et/ou biologique. Cette entité peut être choisie parmi des actifs thérapeutiques chimiques ou de réactifs chimiques comme par exemple un catalyseur ou d'actifs biochimiques en particulier choisis parmi des acides nucléiques comme par exemple l'ARNsi, l'ARNi et l'ADN, des protéines, des peptides, des enzymes, des virus, des bactéries et/ou des cellules.

Selon le procédé de l'invention, les gouttelettes sont chargées avec au moins une entité d'intérêt et l'hydrogel réticulé formé à partir des gouttelettes est isolé des gouttelettes non réticulées.

Selon un mode de réalisation particulier, l'hydrogel réticulé peut être isolé des gouttelettes non réticulées par déstabilisation sélective de celles-ci. Cette déstabilisation sélective peut en particulier être réalisée par mise en contact de l'hydrogel réticulé, en mélange avec les gouttelettes non réticulées, avec au moins un tensioactif. Ce dernier va déstabiliser sélectivement les gouttelettes non réticulées.

Comme il ressort des exemples figurant ci-après les hydrogels, en l'occurrence les microgels obtenus selon l'invention, en considérant notamment des mélanges spécifiques de Dex-Met et notamment ceux détaillés ci-dessus, possèdent bien les propriétés attendues, à savoir une stabilité aux tensio-actifs combinée à une dégradabilité par au moins une enzyme.

Ces enzymes peuvent être génériques à certaines familles de polymères (par exemple les estérases, les métalloprotéases, ...) ou spécifiques (l'alginate lysase pour l'alginate, la hyalunoridase pour l'acide hyaluronique, la dextranase pour le dextran...).

Ainsi et comme illustré dans les exemples qui suivent, la combinaison de 75% en poids du mélange de polymères de DexMet (DS=13%+/-0,5%) et 25% en poids du mélange de polymères de Dex-Met (DS=31% +/-1%), conduit à un microgel à la fois stable et dégradable par la dextranase dans des conditions expérimentales convenant à l'activité catalytique de cette enzyme.

Il est en outre à remarquer qu'une telle combinaison de DexMet conduit à un microgel stable et dégradable correspondant à un DS moyen de 17,5% (correspondant à (0.75x 0.13) + (0.25 x 0.31)). Or, comme illustré également dans les exemples, un microgel obtenu à partir d'un unique Dex-Met de DS variant de 17% à 18% n'est lui ni stable, ni dégradable.

En conséquence, la présente invention permet bien d'accéder via des combinaisons spécifiques de deux types de biopolymères à réticuler simultanément à de nouveaux hydrogels dotés des propriétés attendues en termes de dégradabilité enzymatique et stabilité chimique et non accessibles à partir d'un unique biopolymère.

## Applications

Comme il ressort de ce qui précède, les hydrogels conformes à l'invention sont efficaces pour encapsuler et libérer une ou plusieurs entités d'intérêt.

Ainsi, un des aspects de l'invention concerne également l'utilisation de gouttelettes aqueuses, notamment formées par voie microfluidique, et comprenant au moins un mélange d'au moins deux polydextrans réticulables et chimiquement modifiés notamment avec des motifs choisis thiol, divinylsulfonyle, maléimide, azoture, alcynyle, alcènyle, acrylate, méthacrylate, aldéhyde, norbornènyle, oxy-amine, amine, ester N-hydroxysuccinimide et leurs mélanges, en particulier parmi les acrylates et méthacrylates avec l'un polydextrans réticulables et en particulier un seul d'entre eux possédant un DS inférieur ou égal à 20%, pour l'encapsulation d'au moins une entité d'intérêt notamment telle que définie ci-dessus. Les hydrogels conformes à l'invention peuvent être également utilisés pour la culture cellulaire ou bactérienne notamment en 2D ou en 3D.

L'invention va maintenant être décrite au moyen des figures et exemples suivants, donnés bien entendu à titre illustratif et non limitatif de l'invention.

## Matériels et méthodes

## Matières premières :

Dextran T70 (Dex, Mw = 70 000 g/mol, Pharmacosmos),
Anhydride méthacrylique (94%),
Lithium phenyl-2,4,6-trimethylbenzoylphosphinate (LAP, $\leq$ 95%),
Tampon « phosphate buffer saline » (PBS) (1X : 137 mM NaCl, 2,7 mM KCl, 10 mM Na2HPO4, 1,8 mM KH2PO4 ; pH = 7,4) de Sigma-Aldrich.

Membranes de dialyse (12,000-14,000 Da molecular weight cut-off) de Fisher scientific. Eau désionisée produite avec le purificateur Purelab de ELGA-Veolia (18,2 MOhm.cm). La dextranase est issue de *chaetomiun erraticum* (D0443 chez Sigma-Aldrich).

## Appareillage

Lyophilisateur de Labconco.

Spectromètre Bruker -300 MHz

**Exemple 1**

**Synthèse de dextran-méthacrylé**

Les dextran méthacrylés, de degré de substitution défini, utilisés pour réaliser les microgels ont été synthétisés selon la méthode décrite dans la demande FR 2 114 492.

Le protocole détaillé est comme suit :

Le dextran (5 g) a été dissous dans 100 ml d'eau distillée dans un bécher. Après dissolution complète, différents nombres d'équivalent d'anhydride méthacrylique (de 0,0625 à 0,5 équivalent) par rapport aux groupes hydroxyle du dextran ont été ajoutés goutte à goutte dans la solution de dextran afin d'obtenir le DS souhaité. Ensuite, le pH a été ajusté à l'aide de NaOH (3 mol.l-1) aux alentours de 9-11 pendant toute la durée de la réaction (1h à température ambiante, 20-25°C). Enfin, le polymère Dex-Met obtenu a été dialysé contre de l'eau distillée pendant 1 semaine (membrane 12 000-14 000 Da) avec environ 14 changements de bain pour éliminer les impuretés, et lyophilisé à -46°C, 0,140 mbar pendant 1 semaine. Le solide blanc a été caractérisé par $^1$H RMN et FTIR, et stocké à -20°C avant utilisation.

**Caractérisation du DS**

Il est déterminé à partir des spectres RMN 1H (300 MHz, dans D2O) de chaque biopolymère modifié au regard de celui du biopolymère non modifié, présentés en figure 1.

Les protons typiques de Dextran portés par les carbones C2 à C6 (notés de 2 à 6) donnent un signal de 3,5 à 4,2.

Le proton H1 porté par le carbone C1 donne un signal à 5,1 ppm et 5,4 ppm, noté 1, correspondant respectivement aux liaisons α-1,6 et -1,3.

Une valeur usuelle de 4 % de liaison -1,3 par rapport à la liaison -1,6 a été calculée, sur la base des intégrales des signaux correspondants à 5,4 ppm et des signaux des autres protons de Dex.

Sur les spectres des polymères Dex-Met dialysés et lyophilisés, la disparition des signaux d'acide méthacrylique 5,4 et 5,7 ppm et autres traces d'impuretés indique un polymère bien purifié (>0,01%).

En plus des protons Dex précédemment décrits, des protons du groupe méthacrylate (protons vinyliques notés 9, et protons méthyles notés 10, avec un rapport entre signaux vinyles et méthyles de 2 :3) sont bien visibles sur les spectres des différents Dex-Met, montrant la bonne incorporation des fragments méthacrylate.

Le degré de substitution, défini comme la quantité de groupes méthacrylyle pour 100 résidus de dextran glucopyranose est calculé comme suit

[Math 1]

$$DS = \frac{(I_9 + I_{10}) / 5}{(I_1\,(5,1\;ppm) + I_2 + I_3 + I_4 + I_5 + I_6) / 6,96}$$

Le tableau 1 ci-après détaille les exemples de synthèse de Dext-Met avec différents DS obtenus en fonction de la quantité (nombre d'équivalents) d'anhydride méthacrylique introduite initialement dans le milieu réactionnel.

[Tableau 1]

| Dextran | Anhydride méthacrylique | DS | Rendement de la réaction (%) |
|---|---|---|---|
| 5 g | 0,915 ml - 0,0625 eq | 13% +/- 0,5% | 92 |
| 5 g | 1,83 ml - 0,125 eq | 26% +/- 1% | 90 |
| 5 g | 3,66 ml - 0,25 eq | 52% +/- 2% | 89 |
| 5 g | 4,39 ml - 0,3 eq | 62% +/- 2,5% | 85 |

**Exemple 2**

**Préparation d'hydrogels non conformes à l'invention car à base d'un unique polymère Dext-Met**

**Protocole détaillée de préparation**

La suspension de gouttes est réalisée par un procédé microfluidique de flow focusing utilisant une puce en PDMS commerciale (EZ Drop (DROPKIT01)). Le principe est qu'un écoulement central (polymère) est cisaillé par un écoulement secondaire non miscible orthogonal au premier, formant ainsi des gouttes micrométriques monodisperses. Les écoulements sont contrôlés par un générateur de pression Fluigent. La taille des gouttes est déterminée par les grandeurs géométriques de la puce et par les pressions appliquées. Les microgouttes sont produites à une fréquence comprise entre 300 et 5000 Hz en fonction des pressions appliquées. La taille des microgouttes est autour de 45 $\mu$m de diamètre. La suspension est récoltée dans un tube à centrifuger de 1 ml. Elle est composée de microgouttes de polymères en suspension dans l'huile.

Une solution de polymère Dex-Met (5% poids/poids de Dex-Met, 0,1% poids/poids de LAP dans le PBS 1X) est préparée juste avant la formation des gouttes. Le fluide non miscible est de l'huile mélangée au surfactant (surfactant DSurf à 2% dans l'huile perfluorée 3M™ Novec™ 7500) pour éviter la coalescence des gouttes formées. La puce est positionnée sous un microscope et une caméra rapide permet l'acquisition des images lors de la formation des gouttes. Une protection opaque à la lumière est utilisée pour protéger le polymère et les gouttes formées, photosensibles dans le cas du Dex-Met.

Une fois formulée sous forme de microgouttes, la solution de polymère Dex-Met (5% poids/poids) comprenant le photo-initiateur LAP (Sigma-Aldrich) 0,1% poids/poids est photoréticulée comme suit afin de conduire aux microgels.

40 $\mu$l de la suspension de gouttes sont déposés dans une cuvette formée par deux épaisseurs de Gene Frame (Thermo Scientific™ Cadre génétique, 25 $\mu$l (1,0 x 1,0 cm)) de 0,25 mm d'épaisseur chacune (soit 0,5 mm en tout) collées sur une lamelle de verre. La polymérisation des gouttes de polymère est réalisée par une exposition UV (405 nm) à 75mW/cm$^2$ (lampe UVA-Hand de Honle) pendant 30 secondes. La lamelle exposée 30 secondes aux UV est placée sous le microscope pour la visualisation. 20 $\mu$l de surfactant (1*H*,1*H*,2*H*,2*H*-perfluoro-1-octanol (Sigma-Aldrich)) sont ajoutés dans la cuvette et l'évolution de la suspension est enregistré sur la caméra rapide.

Des microgels de Dex-Met de DS défini (DS = 13% ; 15% ; 17% ; 18% ; 20% ; 25% ; 31% ; 37%) ont été préparés et photoréticulés en reproduisant ce protocole.

**Détermination de leur stabilité et dégradabilité**

La stabilité aux tensioactifs est évaluée en ajoutant à une suspension dans l'huile (40 $\mu$L de solution) du polymère réticulé considéré, 20$\mu$L du 1*H*,1*H*,2*H*,2*H*-perfluoro-1-octanol (Sigma-Aldrich). La déstabilisation est observée sous microscope. Comparativement, il a été noté que la déstabilisation des gouttelettes de polymère non réticulé est très rapide (< 5 minutes).

La dégradabilité est évaluée vis-à-vis de la dextranase. La dextranase est capable d'hydrolyser les liaisons (1->6)-alpha-D-glucosidiques du dextran.

Pour tester la dégradation par la dextranase des microgels réticulés, 5 $\mu$L de dextranase (issue de chaetomiun erraticum, D0443 chez Sigma-Aldrich) sont ajoutés à 20 $\mu$L de solution de gouttes photoréticulées de 45 $\mu$m de diamètre, soit environ 250 000 gouttes., portée à une température de 37°C. La digestion est évaluée par visualisation des gouttes sous microscope. Le Tableau 2 ci-après résume les résultats obtenus avec les microgels issus de la photoréticulation d'un unique type de dextran-méthacrylé.

[Tableau 2]

| Microgel | Stabilité à l'ajout de surfactant | Dégradabilité par la dextranase |
|---|---|---|
| DS=13% | Non | 30 min |
| DS=15,5% | Non | 20 min |
| DS=17% | Non | <1h |
| DS=18% | Non | <1h |
| DS=20% | Oui | 4h30 |
| DS=25% | Oui | > 48 h |
| DS=31% | Oui | > 48 h |

(suite)

| Microgel | Stabilité à l'ajout de surfactant | Dégradabilité par la dextranase |
|----------|-----------------------------------|---------------------------------|
| DS=37% | Oui | > 48 h |

De ces résultats, il ressort que pour un DS supérieur ou égal à 20%, les microgels résistent à l'ajout du surfactant : le réseau polymère est suffisamment solide. En revanche, pour un DS plus faible, les microgels se déstabilisent à l'ajout du surfactant.

Par ailleurs, les microgels réticulés sont digérés en moins de 2h seulement lorsque le DS est inférieur ou égal à 20%. Pour un DS supérieur, l'enzyme ne peut dégrader les microgels, ou alors bien trop lentement (plus de 2h).

Aucun de ces microgels ne permet donc de donner simultanément satisfaction en termes de stabilité et de biodégradabilité contrôlée. Il n'y a en effet pas de microgel qui soit à la fois stable à l'ajout du surfactant et dégradable en moins de 2h par la dextranase à 37°C.

## Exemple 3

### Préparation d'hydrogels à partir de plusieurs mélanges de deux dextran méthacrylés dont un dextran à DS inférieur à 20%

Plusieurs mélanges de dextran méthacrylés en proportion 75% en poids de dextran méthacrylé à DS inférieur à 20%et 25% en poids de dextran méthacrylé à DS supérieur sont réalisés.

Chaque mélange 75:25 de dextran méthacrylé en poudre est dissous en solution aqueuse (eau ou tampon PBS) puis additionné de LAP afin d'obtenir au total en poids pour la phase aqueuse 5% de dextran-méthacrylé et 0,1% de LAP. Cette solution de polymères est ensuite utilisée pour former des microgouttes qui sont photoréticulées en hydrogels selon le protocole détaillé en exemple 2.

La stabilité des hydrogels réticulés ainsi obtenus est testée en présence de surfactant dans la phase huileuse et leur dégradabilité par la dextranase est évaluée comme décrit à l'exemple 2.

Les résultats obtenus sont résumés dans le Tableau 3 ci-après.

[Tableau 3]

| Hydrogel | | | Stabilité à l'ajout de surfactant | Dégradabilité par la dextranase CE |
|----------|---|---|-----------------------------------|------------------------------------|
| 75% en poids d'un faible DS de valeur | 25% en poids d'un fort DS de valeur | DS moyen pondéré | | |
| 13% | 31% | 17,% | Oui | 1 h |
| Unique dextran de DS=18% pur | | | Non | 1 h |
| Unique dextran de DS=20% pur | | | Oui | 4h30 |
| 15,5% | 37% | 20,8% | Oui | 1 h |

De ces résultats, il ressort que seuls les microgels conformes à l'invention donnent satisfaction. Ils permettent en particulier d'obtenir une plage de DS moyen pondéré dans laquelle il est constaté simultanément une stabilité et une dégradabilité contrôlée à moins de deux heures des microgels.

Un microgel, obtenu à partir d'un unique polymère dont la valeur de DS est proche de la valeur de DS moyen pondéré des microgels selon l'invention est dénué soit de la stabilité soit de la dégradabilité attendue.

## Exemple 4

### Préparation d'un hydrogel à partir d'un mélange 50/50 de deux dextrans méthacrylés dont un dextran à DS inférieur à 20%.

Un mélange 50:50 de deux dextrans méthacrylés, l'un de DS = 13% (faible DS) et l'autre de DS = 31% (fort DS) en poudre est dissous en solution aqueuse (eau ou tampon PBS) puis additionné de LAP afin d'obtenir au total en poids pour la phase aqueuse 5% de dextran-méthacrylés et 0,1% de LAP. Cette solution de polymères est ensuite utilisée pour former des microgouttes qui sont photoréticulés en microgels comme décrit à l'exemple 2. La stabilité de ces microgels en présence de surfactant dans la phase huileuse et leur dégradabilité par la dextranase est évaluée comme décrit à l'exemple 2. Les résultats obtenus sont résumés dans le Tableau 4 ci-après qui rappelle en outre les résultats obtenus avec ce même mélange de dextran méthacrylé mais dans une proportion de 75/25 et énoncés en exemple 3.

[Tableau 4]

| Hydrogel | | | Stabilité à l'ajout de surfactant | Dégradabilité par la dextranase CE |
|---|---|---|---|---|
| Qté en Faible DS (DS = 13%) | Qté en Fort DS (DS = 31%) | Valeur DS moyen pondéré | | |
| 50% | 50% | 22% | Oui | 2h |
| Unique dextran de DS à 20% pur | | | Oui | 4h30 |
| 75% | 25% | 17,5% | Oui | 1 h |

De ces résultats, il ressort que le mélange 50:50 DS (DS = 13%), et DS (DS= 31%) reproduit les deux qualités attendues à l'image du mélange 75 :25 faible DS (DS = 13%), et fort DS (DS= 31%).

## Revendications

1. Procédé de préparation d'un hydrogel réticulé à dégradabilité contrôlée comprenant au moins les étapes consistant à

    (a) Disposer de gouttelettes aqueuses comprenant au moins un mélange d'au moins deux biopolymères réticulables et de degrés de substitution, DS différents,
    (b) Exposer lesdites gouttelettes à un stimuli de réticulation efficace pour réaliser la réticulation simultanée des deux biopolymères et,
    (c) Récupérer l'hydrogel réticulé,

    dans lequel l'un des biopolymères possède un degré de substitution inférieur ou égal à 20%

2. Procédé selon l'une quelconque des revendications précédentes dans lequel lesdits biopolymères sont chimiquement modifiés et notamment substitués par des motifs réticulables notamment choisis parmi les motifs thiol, divinylsulfonyle, maléimide, azoture, alcynyle, alcènyle, acrylate, méthacrylate, aldéhyde, norbornènyle, oxy-amine, amine, ester N-hydroxysuccinimide et leurs mélanges, en particulier parmi les acrylates et méthacrylates.

3. Procédé selon l'une quelconque des revendications précédentes dans lequel lesdits biopolymères dérivent de composés choisis les alginates, l'acide hyaluronique, la cellulose, carboxyméthylcellulose, le chitosan, le dextran et en particulier le dextran.

4. Procédé selon l'une quelconque des revendications précédentes dans lequel lesdits biopolymères ne se différencient que par leur degré de substitution, DS.

5. Procédé selon l'une quelconque des revendications précédentes dans lequel lesdites gouttelettes contiennent un mélange d'au moins deux polydextrans de poids moléculaire identique et chimiquement modifiés par des motifs choisis parmi les acrylate, méthacrylate, alcényle, alcynyle et leurs mélanges, en particulier un mélange de deux polydextrans de poids moléculaire identique chimiquement modifiés par des motifs méthacrylate, DexMet, et de DS variant de 10 à 40%.

6. Procédé selon l'une quelconque des revendications précédentes dans lequel lesdites gouttelettes contiennent un mélange d'au moins un DexMet de DS inférieur à 20% et d'un DexMet de DS supérieur à 25% et notamment un mélange de DexMet à DS égal à 13% +/- 0,5% et de DexMet à DS égal à 31% +/-1%.

7. Procédé selon l'une quelconque des revendications précédentes dans lequel lesdites gouttelettes contiennent un mélange à 75% en poids de DexMet à DS égal à 13% +/-0,5% et 25% en poids de DexMet à DS égal à 31% +/-1% ou un mélange 50/50 en poids de DexMet à DS égal à 13% +/-0,5% et 25% en poids de DexMet à DS égal à 31% +/-1%.

8. Procédé selon l'une quelconque des revendications précédentes dans lequel la réticulation est réalisée par voie photochimique.

9. Procédé selon l'une quelconque des revendications précédentes dans lequel lesdites gouttelettes contiennent également au moins un agent réticulant et notamment un photo-initiateur en particulier le phenyl-2,4,6-trimethylbenzoylphosphinate de lithium ou le 1-[4-(2-Hydroxyethoxy) -phenyl]-2-hydroxy-2-methyl-1-propane-1-one.

10. Procédé selon l'une quelconque des revendications précédentes dans lequel lesdites gouttelettes sont formées par voie microfluidique.

11. Procédé selon l'une quelconque des revendications précédentes dans lequel lesdites gouttelettes possèdent une taille moyenne en nombre variant de 10 à 100 $\mu$m et en particulier de 30 à 50 $\mu$m.

12. Procédé selon l'une quelconque des revendications précédentes dans lequel lesdites gouttelettes contiennent au moins une entité d'intérêt chimique et/ou biologique notamment choisie parmi des actifs thérapeutiques chimiques, des réactifs chimiques et des actifs biochimiques en particulier choisis parmi des acides nucléiques tels que l'ARNsi, l'ARNi et l'ADN, des protéines, des peptides, des enzymes, des virus, des bactéries et/ou des cellules.

13. Procédé selon l'une quelconque des revendications précédentes dans lequel lesdites gouttelettes sont chargées avec au moins une entité d'intérêt en particulier telle que définie en revendication 12, et en ce que ledit hydrogel réticulé est isolé des gouttelettes non réticulées par déstabilisation sélective de celles-ci.

14. Procédé selon la revendication précédente dans lequel la déstabilisation sélective est réalisée par mise en contact de l'hydrogel réticulé, en mélange avec les gouttelettes non réticulées, avec au moins un tensioactif.

15. Procédé selon l'une quelconque des revendications précédentes dans lequel l'hydrogel formé est un macrogel, microgel ou nanogel et de préférence un microgel.

16. Hydrogel réticulé à dégradation contrôlée obtenu par le procédé selon l'une quelconque des revendications précédentes.

17. Hydrogel réticulé à dégradation contrôlée, notamment obtenu par le procédé selon l'une quelconque des revendications 1 à 15, **caractérisé en ce qu'**il dérive de la réticulation simultanée d'au moins deux polydextrans chimiquement modifiés avec des motifs choisis parmi les motifs thiol, divinylsulfonyle, maléimide, azoture, alcynyle, alcènyle, acrylate, méthacrylate, aldéhyde, norbornènyle, oxy-amine, amine, ester N-hydroxysuccinimide et en particulier parmi les acrylates et méthacrylates, dont l'un possédant un DS inférieur ou égal à 20%, en particulier de deux polydextrans ne se différenciant que par leur DS et plus particulièrement de deux polydextrans chimiquement modifiés par des motifs méthacrylate, DexMet, et de DS variant de 10% à 40%.

18. Hydrogel selon la revendication précédente dérivant de la réticulation d'un mélange à 75% en poids de DexMet à DS égal à 13% +/-0,5% et 25% en poids de DexMet à DS égal à 31% +/-1% ou d'un mélange 50/50 en poids de DexMet à DS égal à 13%+/- 0,5% et de DexMet à DS égal à 31%+/-1%.

19. Hydrogel selon la revendication 17 ou 18 contenant au moins une entité d'intérêt notamment choisie parmi des actifs thérapeutiques chimiques, des réactifs chimiques et des actifs biochimiques en particulier choisis parmi des acides nucléiques tels que l'ARNsi, l'ARNi et l'ADN, des protéines, des peptides, des enzymes, des virus, des bactéries, des cellules et leurs mélanges.

20. Utilisation d'un hydrogel obtenu selon l'une quelconque des revendications 1 à 16 ou selon l'une des revendications 17 à 20 pour la culture cellulaire ou bactérienne.

21. Utilisation de gouttelettes aqueuses, notamment formées par voie microfluidique, et comprenant au moins un mélange d'au moins deux polydextrans réticulables et chimiquement modifiés avec des motifs choisis parmi les motifs thiol, divinylsulfonyle, maléimide, azoture, alcynyle, alcènyle, acrylate, méthacrylate, aldéhyde, norbornènyle, oxy-amine, amine, ester N-hydroxysuccinimide et leurs mélanges en particulier parmi les acrylates et méthacrylates avec l'un d'entre eux et en particulier un seul d'entre eux possédant un DS inférieur ou égal à 20%, pour l'encapsulation d'au moins une entité d'intérêt. notamment telle que définie en revendication 19.

[Fig 1]

Fig. 1

Scale: 0.4587 ppm/cm, 137.7 Hz/cm

Europäisches
Patentamt

European
Patent Office

Office européen
des brevets

**RAPPORT DE RECHERCHE EUROPEENNE**

Numéro de la demande

EP 24 18 6518

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | STENEKES ROBERT J.H. ET AL: "The use of aqueous PEG/dextran phase separation for the preparation of dextran microspheres", INTERNATIONAL JOURNAL OF PHARMACEUTICS, vol. 183, no. 1, 1 juin 1999 (1999-06-01), pages 29-32, XP093119248, NL ISSN: 0378-5173, DOI: 10.1016/S0378-5173(99)00038-1 | 1-3,8-21 | INV. A61L27/20 A61L27/52 A61L27/54 A61L27/58 |
| Y | * le document en entier * | 4-6 | |
| A | | 7 | |
| | ----- | | |
| Y | EP 4 201 966 A1 (COMMISSARIAT ENERGIE ATOMIQUE [FR]; CENTRE NAT RECH SCIENT [FR] ET AL.) 28 juin 2023 (2023-06-28) * le document en entier * | 4-6 | |
| | ----- | | |
| A | US 2022/118124 A1 (YANG SEUNG YUN [KR] ET AL) 21 avril 2022 (2022-04-21) * exemples * | 1-21 | |
| | ----- | | DOMAINES TECHNIQUES RECHERCHES (IPC) |
| A | DE GEEST BRUNO G. ET AL: "Synthesis of Monodisperse Biodegradable Microgels in Microfluidic Devices", LANGMUIR , vol. 21, no. 23 1 novembre 2005 (2005-11-01), pages 10275-10279, XP093118487, US ISSN: 0743-7463, DOI: 10.1021/la051527y Extrait de l'Internet: URL:https://web.mit.edu/Thorsen/www/Micros pheres.pdf * le document en entier * | 1-21 | A61L A61K |
| | ----- | | |
| | -/-- | | |

Le présent rapport a été établi pour toutes les revendications

| Lieu de la recherche | Date d'achèvement de la recherche | Examinateur |
|---|---|---|
| La Haye | 18 novembre 2024 | Derrien, Anne-Cécile |

Europäisches
Patentamt

European
Patent Office

Office européen
des brevets

# RAPPORT DE RECHERCHE EUROPEENNE

Numéro de la demande

EP 24 18 6518

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | KIM SIN-HEE ET AL: "In Vitro Release Behavior of Dextran-Methacrylate Hydrogels Using Doxorubicin and other Model Compounds", JOURNAL OF BIOMATERIALS APPLICATIONS. , vol. 15, no. 1 27 juillet 2000 (2000-07-27), pages 23-46, XP093118495, US ISSN: 0885-3282, DOI: 10.1106/ML30-FMLG-1HRU-7C4B Extrait de l'Internet: URL:http://journals.sagepub.com/doi/pdf/10.1106/ML30-FMLG-1HRU-7C4B * le document en entier * ----- | 1-21 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| La Haye | 18 novembre 2024 | Derrien, Anne-Cécile |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................................
& : membre de la même famille, document correspondant

EPO FORM 1503 03.82 (P04C02)

**EP 4 487 877 A1**

## ANNEXE AU RAPPORT DE RECHERCHE EUROPEENNE
## RELATIF A LA DEMANDE DE BREVET EUROPEEN NO.

EP 24 18 6518

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-11-2024

| Document brevet cité au rapport de recherche | | Date de publication | Membre(s) de la famille de brevet(s) | | Date de publication |
|---|---|---|---|---|---|
| EP 4201966 | A1 | 28-06-2023 | EP | 4201966 A1 | 28-06-2023 |
| | | | FR | 3131316 A1 | 30-06-2023 |
| US 2022118124 | A1 | 21-04-2022 | CN | 113366026 A | 07-09-2021 |
| | | | EP | 3919521 A1 | 08-12-2021 |
| | | | JP | 7431838 B2 | 15-02-2024 |
| | | | JP | 2022519093 A | 18-03-2022 |
| | | | KR | 20200095157 A | 10-08-2020 |
| | | | US | 2022118124 A1 | 21-04-2022 |
| | | | WO | 2020159080 A1 | 06-08-2020 |

EPO FORM P0460

Pour tout renseignement concernant cette annexe : voir Journal Officiel de l'Office européen des brevets, No.12/82

16

**RÉFÉRENCES CITÉES DANS LA DESCRIPTION**

**Documents brevets cités dans la description**

- FR 2114492